(19) Europäisches Patentamt
European Patent Office
Office européen des brevets

(11) **EP 2 907 498 A1**

(12) **EUROPEAN PATENT APPLICATION**

(43) Date of publication:
**19.08.2015 Bulletin 2015/34**

(51) Int Cl.:
***A61K 8/73*** *(2006.01)*     ***A61Q 19/00*** *(2006.01)*
***A61K 8/02*** *(2006.01)*

(21) Application number: **14305185.2**

(22) Date of filing: **13.02.2014**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **INDUCHEM AG**
**8604 Volketswil (CH)**

(72) Inventors:
 • **Schweikert, Kuno**
  **CH-8808 Pfäffikon SZ (CH)**

 • **Egorova, Marina**
  **8052 Zurich (CH)**
 • **Juch, Rudolf-Dieter**
  **8650 Barão de São Miguel (PT)**
 • **Lefevre, Fabrice**
  **31190 Auterive (FR)**
 • **Westenfelder, Horst**
  **67435 Neustadt (DE)**

(74) Representative: **Lebrette, Camille et al**
**BECKER & ASSOCIES**
**25, rue Louis Le Grand**
**75002 Paris (FR)**

(54) **Exfoliating cellulose beads and cosmetic uses thereof**

(57)    The present invention relates to cellulose particles with measurable exfoliation properties in a cosmetic composition, and uses thereof in the cosmetic field.

**EP 2 907 498 A1**

## Description

The present invention relates to the cosmetic and dermocosmetic field.

## PRIOR ART

The skin of the human body self-renews continuously by eliminating the upper, external, layers and generating new internal layers from the basal layer. Desquamation normally occurs invisibly with shedding of dead cells resulting from desquamation. During natural desquamation process, the skin can shed spontaneously 30,000 to 40,000 dead cells per minute. However, disturbances in this process result in the accumulation on the skin surface of only partially detached cells with or without a concomitant thickening of the stratum corneum. Such cells and debris accumulation on the skin surface is generally combined with a feeling of roughness and dryness of the skin surface and may lead to flaky skin, less soft and smooth, non-uniform skin color, etc.

Exfoliation of dead cells and debris from the epidermis is a well-known cosmetic process that generates abrasive tensions on skin surface, thereby removing dead cells and promoting regeneration of the epidermal tissue. Additional potential benefits to exfoliation are improved skin cleansing by helping to mechanically remove dirt and oil from the skin, reduction in bacteria on the skin and increased blood flow to the skin due to the mechanical stimulation. Exfoliation can be accomplished by the use of natural sponge or rag, or exfoliating compositions.

Exfoliating compositions generally comprise abrasive particles to facilitate removal of the cells and debris. Known abrasive particles include fruit kernel fragments, in particular apricot and cherry, almond or nut shell fragments, sand, pearl fragments or plastic particles, in particular polyethylene beads.

The use of exfoliating compositions has been associated with an increase of skin suppleness and softness. Penetration of cosmetic is also facilitated. However, the majority of the natural abrasive particles are not gentle enough for a daily use and/or for use on sensitive skins. Indeed, they tend to induce skin irritations due to their too drastic mechanical effect and to the micro-cuts they may cause. Furthermore, the plastic abrasive particles, even if they exhibit lower drawbacks are considered as non-sustainable and polluting.

Therefore, there is a need for improved abrasive particles and exfoliating composition for the application in personal care and cosmetics, based on natural resources.

## SUMMARY OF THE INVENTION

By conducting experiments and researches on cellulose particles, the inventors have discovered that particular particles may exhibit same exfoliating properties as fruit kernel particles without their associated drawbacks. Furthermore, the inventors have surprisingly found that these cellulose particles, or cellulose beads, get destroyed during skin application so that they naturally disappear from the body surface without any rinsing. These cellulose particles can be advantageously used in a body care composition, and notably for exfoliating composition.

It is therefore an object of the invention to provide cellulose particles having an average particle size less than or equal to 2 mm, wherein the cellulose particles provide measurable exfoliation after 1 day in a cosmetic composition, preferably after 3 months in in a cosmetic composition, more preferably after 6 months in a cosmetic composition, even more preferably after more than 12 months in a cosmetic composition. In a particular embodiment, the cellulose particles provide measurable exfoliation after 30 months at room temperature in a cosmetic composition.

Advantageously, at least 80%, preferably at least 90%, more preferably at least 95%, and even more preferably at least 99% of the cellulose particles after 1 day in a cosmetic composition, disintegrate towards cellulose powder upon applying. Preferably, the cellulose particles disintegrate after an average time comprised between 10 seconds and 3 minutes.

In a particular embodiment, the cellulose particles contain at least one cosmetic agent and/or color agent, preferably a liposoluble agent. For instance, the cosmetic/color agent is chosen among vitamins, coenzymes, essential oils, natural oils, silicone oils, clay or metal or precious stone powder, D-panthenyl triacetate and pigments.

The present invention further relates to the cosmetic use of such cellulose particles as exfoliating agents for skin or scalp. For instance, the cellulose particles may be used to exfoliate during cleansing of the skin or scalp of the body, and/or to exfoliate part of body skin or scalp without rinsing out.

In another aspect, the invention relates to the use of said cellulose particles for the preparation of a body care composition, in particular cleansing body composition, hair care composition and cosmetic composition.

It is therefore an object of the invention to provide with a body care composition, comprising cellulose particles of the invention.

According to the invention, the cellulose particles of the body care composition provide measurable exfoliation after 3 months storage at room temperature (i.e. around 20°C to 25°C), preferably after 6 months, more preferably after 12 months storage at room temperature, even more preferably after 30 months storage at room temperature.

Advantageously, at least 80%, preferably at least 90%, more preferably at least 95%, and even more preferably at least 99% of the cellulose particles of the body care composition disintegrate after an average playtime less than 3 minutes, preferably less than 2 minutes and more preferably less than 1 minutes, when applied on human skin or scalp.

In a particular embodiment, the body care composition comprises 0.5% to 20% by wt. of said cellulose particles, preferably 1% to 15% by wt., more preferably 2% to 10% by wt., even more preferably 3% to 5% by wt.

Advantageously, the distribution size of the cellulose particles in the composition ranges between 0 and 200 $\mu$m, or between 200 and 400 $\mu$m, or between 400 and 900 $\mu$m.

Preferably, the cosmetic composition of the invention comprises at least 10 % by weight relative to the total weight of the composition of water, for instance at least 15, 20, 25, 30, 35, 40, 45, 50, 60, 70, 80 or 90 % by weight relative to the total weight of the composition of water.

According to the invention, the body care composition is for instance a cleansing body composition, in particular a peeling-shower gel composition, a hair care composition, in particular a peeling-shampoo, or a cosmetic composition, in particular exfoliating gels, whitening and/or brightening composition, body and feet scrub composition.

The composition of the invention may be used for instance for preventing and/or reversing the sign of skin aging and/or improving skin smoothness and softness and/or recovering skin moisturization and/or preventing trans-epidermal water loss in skin.

In an embodiment, the body care composition further comprises at least one additive selected from the group consisting of excipients, vehicle ingredients, moisturizers, humectants, cosmetic salts, adjuvants, oils, emulsifiers, co-emulsifiers, gelling agents, absorbers, solvents, photo-protective agents, and inert base.

The present invention further relates to a cosmetic method of exfoliating skin or scalp, comprising applying to said skin or scalp a composition as described above.

## BRIEF DESCRIPTION OF THE DRAWINGS

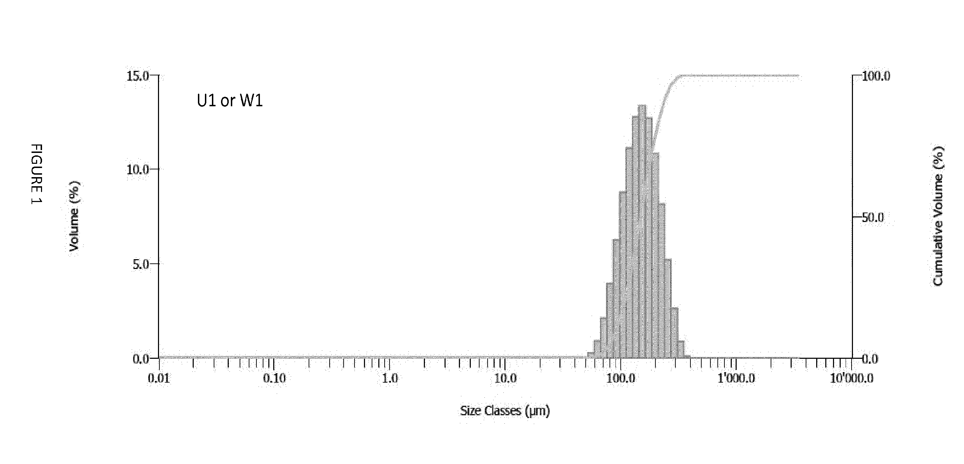

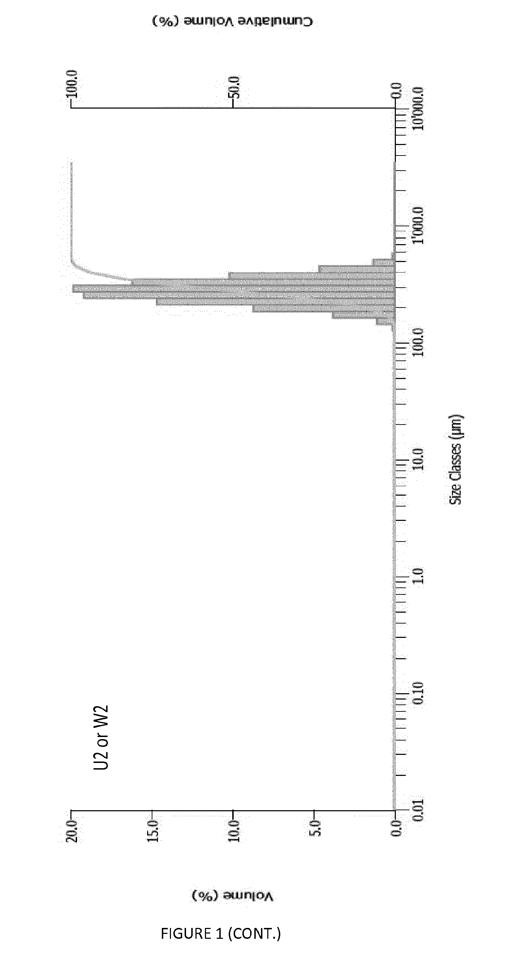

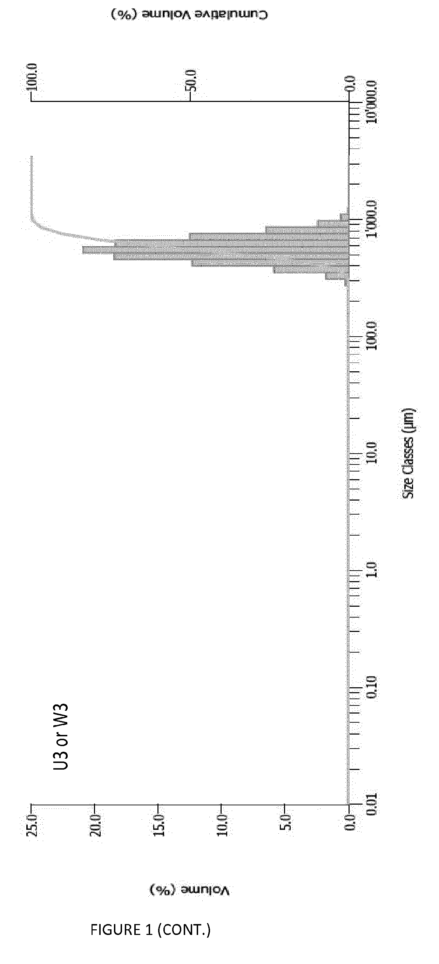

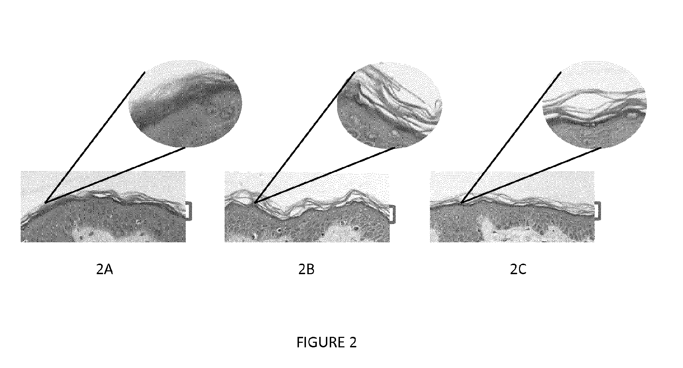

Figure 1 shows the three distribution sizes of cellulose particles, respectively 0-200 $\mu$m, 200-400 $\mu$m and 400-900 $\mu$m. Figure 2 shows histological observations of untreated skin (control - figure 2A), skin treated with apricot exfoliator (figure 2B) and skin treated with cellulose particles of the invention (figure 2C) obtained by staining the skin sample with Hemalun Eosine.

## DETAILED DESCRIPTION OF THE INVENTION

The features and advantages of the present invention will be more readily understood, from reading the following detailed description. It is to be appreciated those certain features of the invention, which are, for clarity, described above and below in the context of separate embodiments, may also be provided in combination in a single embodiment. Conversely, various features of the invention that are, for brevity, described in the context of a single embodiment, may also be provided separately or in any sub-combination.

In the context of the invention, "exfoliation", "peeling" or "scrub" may be used indifferently to refer to the action of removing dead cells from skin or scalp.
In the same way, "cellulose particles" and "cellulose beads" are used interchangeably to designate agglomerates of compacted microcrystalline cellulose.

*Cellulose particles*

By conducting particular research on cellulose, the inventors have discovered that it is possible to produce porous cellulose particles showing exfoliating properties even after long time storage in a cosmetic formulation. These particles are obtained from a mixture composed of cellulose and water, optionally in combination with pigments and/or aid materials as hydroxypropyl-methylcellulose, acrylates/ammonium methacrylate copolymer and triethylcitrate

More particularly, unlike the cellulose particles of the prior art that lost their exfoliating properties once they are put in a cosmetic composition, the cellulose particles of the invention provide measurable exfoliation after 1 day in a cosmetic composition at room temperature, and more preferably even after 3, 6, 12 or 30 months in a cosmetic composition at room temperature.

Advantageously, the cellulose particles, when contain in a cosmetic composition, are spreadable and disintegrate towards fine cellulose powder upon application on the skin. Indeed, the inventors have surprisingly discovered that at least 80 %, preferably at least 90%, more preferably at least 95%, even more preferably at least 99% of the beads disintegrate under mechanical stress or during massage movements after an average time less than 3 minutes, when applied on human skin or scalp. The disappearing properties, or disintegrating properties, of the cellulose particles may be assessed by a "play time" test wherein volunteers apply different cosmetic composition samples and determine the application time when particles completely disintegrate and disappear on skin. The "play time" may depend on the size of the cellulose particles and on the concentration of said cellulose particles in the composition. The larger the particles and the higher the concentration, the longer time it is to obtain complete disintegration of the cellulose particles. One skilled in the art is able to adapt the play time of the cellulose particles according to the nature and/or destination of the composition.

Otherwise or in addition, the cellulose particles can disintegrate immediately when the pressure applied on them is too intense. Conversely, when the cellulose particles are not manipulated, they remain stable in water or a cosmetic composition for at least 6 months at a temperature of 40°C to 50°C. In more classical conditions of storage, i.e. at a temperature comprised between 5°C and 25°C, the cellulose particles remain stable in water or a cosmetic composition for more than 12 months, and more particularly for at least 30 months.

By "stable", it is intended that the cellulose particles do not disappear or disintegrate, even partially. Stability may be easily assessed visually.

For instance, one may visually notice that a cosmetic composition containing cellulose particles of the invention does not exhibit a change of color or separation phases, even after long time storage. A "long time storage" refers to a storage of at least 6 months, preferably at least 12 months, even more preferably at least 30 months at room temperature (i.e. between 20°C and 25°C). The stability may be further assessed analytically, by measurement of pH or viscosity of the composition: no change would be noticed even after a long time storage. Otherwise, or in addition, the odor of the composition containing cellulose beads of the invention may be compared with the odor of a pure base that does not contain beads. No difference between the two compositions would be noticed, even after a long time storage.

The dissolving properties of the cellulose beads of the invention in a cosmetic composition may have several advantages and applications in cosmetic.

At first, the beads can be loaded with cosmetic/color agents that are delivered at the site of interest when the beads disintegrate. Any suitable cosmetic agent may be used, including vitamins, in particular vitamins A or E, coenzymes, cooling agents, D-panthenyl triacetate, plant extracts, oils, in particular hydrating oils, essential oils, natural oils (e.g.: tea tree oil, jojoba oil, marula oil, argan oil), silicone oils, clay or metal or precious stone powders, pigments, etc. Advantageously, the cosmetic or color agents are liposoluble so that they do not diffuse in a cosmetic composition containing water.

Secondly, as the cellulose particles leave no residues when they disintegrate, there is no need of rinsing out the skin or scalp to remove the particles from the skin. It is of particular interest for hair care formula, like anti-oily dandruffs shampoo, wherein the rinsing time for preventing residues and associated irritations is often time consuming and can be noticeably shortened. In addition, the dissolving properties of the cellulose particles may be further useful to inform the user when the exfoliation is completed.

According to the invention, the cellulose particles have an average particle size less than 1 mm and preferably comprised between 200 $\mu$m and 900 $\mu$m. The distribution size advantageously follows a Gaussian distribution curve and one may easily obtained specific ranges with control sizes. Advantageously, the cellulose particles of the invention have a distribution size range between 0 and 200 $\mu$m, or between 200 and 400 $\mu$m or between 400 and 900 $\mu$m.

*Exfoliation measure tests*

The exfoliation properties of the cellulose beads may be measured after one day in a cosmetic composition, whatever the composition is. In a particular embodiment, the measure of the exfoliation properties may be performed by using water. Advantageously, the measure is performed after 3 months in the composition at room temperature, preferably after 6 months in the composition at room temperature, more preferably after 12 months in the composition at room temperature, even more preferably after 30 months in the composition at room temperature.

One or more of the following tests can be used to characterize the exfoliation properties of the cellulose particles of the invention and compare them to comparative exfoliating means or comparative cellulose particles. Convincing results may be obtained compared to cellulose particles of the prior art from the first day of storage in water or cosmetic composition, since the cellulose particles of the prior art lose their exfoliating properties when they are put in water or cosmetic composition.

For instance, the exfoliation may be measured by assessment of stratum corneum cohesion by staining, wherein

1) Human skin explants would be obtained from a panel of healthy donors (for instance 4-10 healthy donors);
2) The skin explants would be stained;
3) Half the skin explants would be untreated (control) and the other half would be treated for two consecutive days with a composition comprising the cellulose particles of the invention;
4) At the end of the test, the stratum corneum cohesion would be evaluated histologically (on a 0-5 point scale) on the decrease of stratum corneum cohesion.

Alternatively, exfoliation can also be evaluated in a consumer test wherein

1) A panel of men and/or women (for instance 10-30 men and/or women) in the age group of 25-65 and who are cleansing composition users would be recruited;
2) Half the panelists would first use a composition comprising the cellulose particles of the invention for a week, and the other half would use first a comparative composition for a week;
3) Then the panelists would use for a second week the other composition;
4) At the end of the test, the panelist rate their preference (on a 0-5 point scale) on the attribute of "exfoliation".

The degree of exfoliation is defined as the consumer rating on the 0-5 point scale.

Otherwise, exfoliation can also be evaluated according to the d-squame® test, wherein a d-squame® tape is applied on a stained treated skin under uniform pressure for a time comprised between 20 seconds and 60 seconds, preferably 30 seconds, and then removed. The d-squame® tape is then imaged with suitable camera and the images are analyzed to evaluate the intensity of stained cells. By comparing this data to similar information from a stained untreated skin, one can estimate the amount of exfoliation caused by the exfoliating product of interest as follows:

$$\text{Exfoliation} = (\text{area of d-squame® covered by stain on untreated skin} - \text{area of d-squame® covered by stain on treated skin}) / (\text{area of d-squame® covered by stain on unwashed site})$$

*Cellulose particle manufacture*

It is an object of the invention to provide cellulose particles suitable for cosmetic uses that remain hard enough to allow skin exfoliation when included in a cosmetic composition.

In a particular embodiment, the cellulose particles of the invention comprise cellulose and hydroxypropyl-methylcellulose. Advantageously, the particles are composed between 80% and 100% by wt., preferably between 95% and 99.9%, of cellulose, and between 0% and 20% by wt., preferably between 0.1% and 5%, of hydroxypropyl-methyl-cellulose.

In another embodiment, the cellulose particles are composed of cellulose and acrylates/ammonium methacrylate copolymer and triethylcitrate. Advantageously, the particles are composed between 90% and 100% by wt., preferably between 95% and 100%, of cellulose, and between 0% and 10% by wt., preferably between 0.5% and 5%, of acrylates/ammonium methacrylate copolymer and between 0% and 5% by wt., preferably between 0.05% and 2%, of triethylcitrate.

In another embodiment, the cellulose particles are composed of cellulose solely.

Advantageously, the cellulose particles further contained liposoluble and/or hydrosoluble cosmetic agents, such as vitamins, coenzymes, cooling agents, D-panthenyl triacetate, plant extracts, oils, clay or metal or precious stone powders, pigments, etc. Preferably, the total final content of such cosmetic agents in the cellulose particles is between 0% and 10% on a weight basis, and more preferably between 0.5% and 5%.

According to the invention, the cellulose particles may be prepared by mixing all the raw components, preferably in a powder form, together. Water and/or another aqueous component is then added to the powders, and mixed to form a bulk that is further granulated. The wet granulates are dried until the product shows a moisture content value of 2% to 10% on a weight basis, and preferably of 3% to 5%. The bulk may be dried using conventional drying procedure such as air drying, spray drying, belt drying, freeze drying, drum drying or flash drying. Then, the exfoliating properties and optionally the disintegrative properties of the granulates may be assessed by any means. Advantageously, the granulates may be finally sieved in size fractions, and preferably in three size fractions, respectively 0-200 μm, 200-400 μm and 400-900 μm.

*Body care compositions*

The cellulose particles of the invention may be used as exfoliating agent for skin and scalp. For instance, the particles may be used to exfoliate during cleansing of the body, or during an application that does not require to rinse it out. Advantageously, the cellulose particles of the invention are used for the preparation of a body care composition. In an embodiment, the composition comprises 0.5% to 20% by wt. of said cellulose particles relative to the total weight of the composition, preferably 1% to 15% by wt., more preferably 2% to 10% by wt., even more preferably 3% to 5% by wt. When expressed in ppm, the composition typically comprises from 5000 to 200000 ppm of cellulose particles, preferably from 10000 to 150000 ppm, more preferably 20000 to 100000 ppm, still more preferably from 30000 to 50000 ppm.

According to the invention, it is possible to adapt the "play time" of the cellulose particles to the composition.

Indeed, the nature and sensitivity of the skin may vary from an individual to another but also from a part of the body to another part. For instance, the exfoliating power required for a feet scrub composition is generally higher than the exfoliating power of a face peeling composition, and the sensitivity of a baby skin is different from the sensitivity of an adult skin. The size and/or the composition of the cellulose particles may be advantageously adapted to modulate the exfoliating properties of the composition. In a particular embodiment, the distribution size of cellulose particles in the cosmetic composition ranges between 0 and 200$\mu$m with an average size of 100 $\mu$m (upper graph of figure 1). In another embodiment, the distribution size ranges between 200 and 400 $\mu$m with an average size of 300 $\mu$m (middle graph of figure 1), or between 400 and 900 $\mu$m with an average size of 650 $\mu$m (lower graph of figure 1). One skilled in the art is able to adapt the distribution sizes of the cellulose particles in the composition according to the nature and/or destination of the composition.

The body care composition of the invention is for instance a cleansing body composition, in particular a peeling-shower gel composition, or a hair care composition, in particular a peeling-shampoo, or a cosmetic composition, in particular exfoliating gels, whitening and/or brightening composition, body and feet scrub compositions.

The composition can be formulated as a cream, a gel, a moisturizer, a lotion, a milk, an oil, an ointment, a wax, a mousse, a paste, a serum, a pomade. It may also be in solid form, such as in the form of a stick. It may be used as a care product and/or as a makeup product for the skin.

In a preferred embodiment, the cosmetic composition of the invention comprises at least 10 % by weight relative to the total weight of the composition of water, for instance at least 15, 20, 25, 30, 35, 40, 45, 50, 60, 70, 80 or 90 % by weight relative to the total weight of the composition of water.

Cosmetic additives may be added to achieve a desired cosmetic result. Desired cosmetic results may be determined by one of ordinary skill in the art or the user of the disclosed compositions. Cosmetic additives may include, but are not limited to, carriers, excipients, vehicle ingredients, moisturizers, humectants, cosmetic salts, adjuvants, oils, emulsifiers, co-emulsifiers, gelling agents, absorbers, solvents, photo-protective agents, and inert bases.

The compositions for topical application may contain additional ingredients such as carrier, excipient, or vehicle ingredients such as, for example, water, acetone, ethanol, ethylene glycol, propylene glycol, butane- 1,3-diol, isopropyl myristate, isopropyl palmitate, mineral oil, and mixtures thereof to form lotions, tinctures, creams, emulsions, gels or ointments which are non-toxic and pharmaceutically or dermatologically acceptable. Additionally, moisturizers or humectants can be added to the present compositions if desired. Examples of such additional ingredients can be found in Remington's Pharmaceutical Sciences, Eighteenth Edition, A. R. Gennaro, Ed., Mack Publishing Co., Easton, Pa., 1990.

The body care composition of the invention may be in any presentation form normally used in cosmetics, and it may, for example, be in the form of an optionally gelled aqueous solution, a dispersion of the lotion type, optionally a two-phase lotion, an emulsion obtained by dispersing a fatty phase in an aqueous phase (O/W emulsion) or conversely (W/O emulsion), a triple emulsion (W/O/W or O/W/O emulsion), nanoemulsions, in particular O/W nanoemulsions, in which the size of the drops is less than 100 nm; or a vesicular dispersion of ionic and/or nonionic type. These compositions are prepared according to the usual methods.

When the composition is in aqueous form, especially in the form of an aqueous dispersion, emulsion or solution, it may comprise an aqueous phase, which may comprise water, deionized water, a floral water and/or a mineral water.

In a known manner, the composition disclosed herein may also comprise at least one adjuvant chosen from adjuvants that are common in cosmetics, such as hydrophilic and lipophilic gelling agents, hydrophilic and lipophilic active agents, preserving agents, antioxidants, solvents, fragrances, fillers, screening agents, pigments, odor absorbers and dyestuffs. The at least one adjuvant is present in an amount ranging, for example, from 0.01 % to 20% by weight relative to the total weight of the composition. Depending on its nature, the at least one adjuvant may be introduced into the fatty phase, into the aqueous phase, or into lipid vesicles. In any case, these adjuvants, and also the proportions thereof, will be chosen so as not to harm the desired properties of the combination of the other components of the cosmetic formula.

When the body care composition disclosed herein is an emulsion, the proportion of the fatty phase may range from 5% to 50% by weight such as from 5% to 20% by weight relative to the total weight of the composition. The oils, emulsifiers and co-emulsifiers used in the cosmetic composition in emulsion form are chosen from those conventionally used in the field under consideration. The emulsifier and co-emulsifier are present in the composition in an amount ranging from 0.3% to 30% by weight such as from 0.5% to 20% by weight relative to the total weight of the composition. As oils which may be used in this disclosure, mention may be made, for example, of mineral oils (liquid petroleum jelly or hydrogenated polyisobutene), oils of plant origin (avocado oil or soybean oil), oils of animal origin (lanolin), silicone oils (cyclomethicone or dimethicone) and fluoro oils (perfluoropoly ethers). Fatty alcohols (cetyl alcohol), fatty acids and waxes (carnauba wax or ozokerite) may also be used as fatty substances. As examples of emulsifiers and co-emulsifiers that may be used herein, mention may be made, for example, of fatty acid esters of polyethylene glycol such as PEG-100 stearate, and fatty acid esters of glycerol such as glyceryl stearate.

Hydrophilic gelling agents that may be included are, for example, carboxyvinyl polymers (carbomer), acrylic copolymers such as acrylate/alkylacrylate copolymers, polyacrylamides, such as crosslinked polyacrylamido-methylpropane-sulphonic acid, polysaccharides, natural gums and clays, and lipophilic gelling agents that may be mentioned include, for example, modified clays, such as bentones, metal salts of fatty acids, hydrophobic silica and poly ethylenes. In one embodiment, the hydrophilic gelling agent for the composition disclosed herein is chosen from a crosslinked polyacrylamido-methylpropane-sulphonic acid as described in EP 0850642, WO9800094 or the Hostacerin AMPS commercialized by Clariant.

The following are experimentations and examples provided solely to illustrate the present invention and are not intended to limit the scope of the invention, as described herein.

**EXPERIMENTATIONS**

***Experimentation 1: manufacture of cellulose particles of the invention***

a) Raw materials:

- 90% - 100% by wt. of cellulose,
- 0% - 5% by wt. of hydroxypropyl methyl-cellulose,
- 0%-5% by wt. of liposoluble active ingredients (e.g. D-Panthenyltriacetate, Vitamin E) and/or watersoluble extract and/or pigment

are thoroughly mixed in an industrial mixing machine.

After mixing 50% - 75% by wt. of water are added to the dry blend and the mass is mixed again to obtain a wet granulate.

The wet granulate is being dried in a fluid bed dryer at a temperature of 55 -75°C until the residual water content of the product reaches 3%.

The final product is sieved in 3 size fractions: 0-200 $\mu$m, 200-400 $\mu$m and 400-900 $\mu$m.

b) Raw materials:

- 90% - 100% by wt. of cellulose,
- 0% - 5% by wt. of hydroxypropyl-methylcellulose,
- 0%-5% by wt. of liposoluble active ingredients (e.g. D-Panthenyltriacetate, Vitamin E) and/or watersoluble extract and/or pigment

are thoroughly mixed in an industrial mixing machine and then transferred to a fluid bed reactor.

Granulation is performed by spraying 60% - 90% water in the air flow at 55 -75 °C.

The final product is sieved in 3 size fractions: 0-200 $\mu$m, 200-400 $\mu$m and 400-900 $\mu$m.

c) Raw materials:

- 90% - 100% by wt. of cellulose,
- 0% - 5% by wt. of hydroxypropyl-methylcellulose,
- 0%-5% by wt. of liposoluble active ingredients (e.g. D-Panthenyltriacetate, Vitamin E) and/or watersoluble extract and/or pigment

are thoroughly mixed in an industrial mixing machine, reloaded in a slurry reactor and mixed with 80% - 140% water.

The suspension is being spraydried in a fluid bed reactor at 55 -75 °C.

The final product is sieved in 3 size fractions: 0-200 $\mu$m, 200-400 $\mu$m and 400-900 $\mu$m.

d) Raw materials:

- 90 - 98% by wt. of cellulose particles produced according to any of the methods a) to c),
- 0.5 - 5% by wt. of Acrylates/Ammonium Methacrylate Copolymer
- 0.05 - 2%, by wt. of Triethylcitrate
- 0.01 - 3% by wt. of pigment

The cellulose particles are loaded in a fluid bed dryer. The coating suspension is being spraydried at 55 -75 °C. The final product is sieved to obtain the necessary size fraction.

*Experimentation 2*: *Ex vivo assessment of the cellulose particles*

The aim of this *ex vivo* study was to explore the exfoliating property of the cellulose particles of the invention on the human skin explants maintained in survival condition. The general morphology of the skin treated with the cellulose particles in particular epidermis and stratum corneum was observed in comparison to an Apricot kernel.

***Material & method***

Skin explants

Human skin explants were obtained from abdomino-plasty or mammal reduction from 6 healthy donors.

Gel preparations

Table 1: composition of the gels

| Part | Trade Name | INCI Name | Supplier | % w/w |
|---|---|---|---|---|
| A | Water | Aqua | | ad 100 |
| | Glycerin > 85% | Glycerin | | 2.00 |
| | **Unidiacide U-26** | Diazolidinyl Urea | Induchem CH | **0.28** |
| | Carbopol Ultrez 21 | Acrylates/C10-30 Acrylate Crosspolymer | Carbopol Lubrizol USA | 0.40 |
| | | | | |
| A1 | **Unitamuron H-22** | Pentylene Glycol; Tamarindus Indica Seed Gum | Induchem CH | **4.00** |
| | | | | |
| A2 | Butylene Glycol | Butylene Glycol | | 3.00 |
| | **Phenoxyethanol** | Phenoxyethanol | Induchem CH | **0.30** |
| | Parfum "Mango" | Parfum | | 0.02 |
| | Eumulgin L | PPG-1-PEG-9 Lauryl Glycol Ether | Cognis D | 0.80 |
| | | | | |
| B | Sodium Hydroxide 10% | Sodium Hydroxide / Aqua | | 1.00 |
| pH value: 6.5 - 7 | | | | |

Procedure:

1. Disperse A with stirring till homogenous
2. Add Part A1 with stirring.
3. Blend Part A2 and add it with stirring to Part A/A1
4. Neutralize with Part B.

5. Add 5% by wt. of cellulose particles or 5% by wt. of apricot exfoliator slowly with stirring to the finished gel.

Treatments of skin explants

Explants were either

- left untreated (control), or
- treated with the gel of the invention (containing 5% cellulose particles of the invention with a particle size ranging from 400 to 900 $\mu$m), for two consecutive days; or
- treated with the comparative gel (containing 5% apricot exfoliator 500) for two consecutive days.

The products were applied on days D0 and D1 in the mentioned concentrations topically on the basis of 2 mg per cm$^2$, using a small spatula. The products after application were left on skin surface without any cleaning (to avoid stratum corneum shedding due to mechanical cleaning). The culture medium was renewed at D1.

Histological analysis

*Preparation of explants for histological analysis:*

After treatment, at D2, explants were fixed in buffered formalin. After the fixation in formalin, the samples were dehydrated and impregnated in paraffin. The samples were then embedded and sections were made using a microtome. The observation of the general morphology was performed after staining of formalin fixed and paraffinized sections by hemalun eosine.

*Grading of stratum corneum cohesion:*

The assessment of stratum corneum cohesion is directly related to exfoliation activity of the products. Based on skin histological sections, stained with hemalun eosin the anatomopathologist scored the stratum corneum cohesion following a specific grading:

Score 0: no modification of stratum corneum cohesion
Score 1: slight decrease of stratum corneum cohesion
Score 2: moderate decrease of stratum corneum cohesion
Score 3: important decrease of stratum corneum cohesion
Score 4: very important decrease of stratum corneum cohesion

Remark: The cohesion assessment was performed on the whole section (10 to 15 fields at the magnification 40 were analyzed).

***Results***

Assessment of stratum corneum cohesion:

A significant decrease in stratum corneum cohesion is seen in comparison to control skin (not treated) for both products tested (Gel with 5% cellulose particles of the invention and Gel with 5% apricot exfoliator 500) see table 2 below.

Table 2: Histological assessment of stratum corneum cohesion (*: statistically significant difference in comparison to control skin (Student test, $p < 0,05$)).

| Type of condition assessed | Stratum corneum cohesion score (average score) | |
|---|---|---|
| Control skin | $1,7 \pm 0,49$ | |
| Skin + Gel with 5% cellulose particles of the invention | **2,07** $\pm$ 0,45 | * p = 0,003 |
| Skin + Gel with 5% Apricot Exfoliator 500 | **2,10** $\pm$ 0,74 | * p =0,045 |

Histologically, no significant difference was seen between the test product (Gel with 5% cellulose particles of the invention) and the reference product (Gel with 5% apricot exfoliator 500) concerning their desquamation properties. Both products are moderate exfoliators with a more loosing stratum corneum in comparison to the skin untreated (narrowed stratum corneum), as shown figure 2.

One may further note that the skins treated by exfoliator gels were not rinsed after their application. If the skins were rinsed after application, the exfoliation of the stratum corneum will be more pronounced.

*Experimentation 3: Clinical investigation of the cellulose particles*

To confirm the effectiveness of the cellulose particles of the invention as exfoliating particles a clinical investigation was performed with a gel containing them.

The efficacy parameters of exfoliation were assessed based on: skin softness and skin cleanness.

The tolerance parameters were the following: skin redness and skin irritation. These tolerance parameters were followed as the skin exfoliation process often impacts them.

**Material & method**

Products used:

Gel BAE: Gel formula (as disclosed in experimentation 2) further containing 5% apricot exfoliator

Gel BUD: Gel formula (as disclosed in experimentation 2) further containing 5% cellulose particles of the invention with a particle size ranging between 200 and 400 $\mu$m

Panel and study conditions

A panel test was run on 29 people, 20 of them being women, to evaluate and compare BUD composition to BAE composition.

The study was simple blinded. Each tester used BUD composition on one side of their face and BEA composition on the other side of their face. The volunteers were asked to massage their faces with light circular movements then wash the products with water. The products were used for one week at the testers' convenience as many time as desired. The methods used to assess the efficacy of the products are described in detail in the following sections.

Efficacy assessment

Assessment of exfoliating properties (in use tests):

The following criteria were assessed by questionnaire (self-assessments):

- Skin softness sensation after washing
- Skin cleaner sensation after washing
- Product preference

Safety assessment (Tolerance properties)

The tolerance properties of the exfoliating product were assessed during use tests by the following criteria (questionnaire):

- Sensation of skin aggression (feeling of scratches) by exfoliating particles
- Skin redness and slight irritation (after intensive use)

**Results**

Efficacy assessment

*Skin softness sensation after washing*

Table 3: Skin softness assessment (Questionnaire)

| Type of product | Numbers of volunteers | | | % of volunteers | | |
|---|---|---|---|---|---|---|
| | Very soft | Soft | No difference | Very soft | Soft | No difference |
| BAE | 11 | 15 | 1 | 41 | 56 | 3 |
| BUD | 12 | 17 | 0 | 42 | 58 | 0 |

Both products assessed are efficient skin exfoliators. The product BUD has shown the best efficacy in comparison to BAE product. 100% of the volunteers found their skins (soft or very soft) after the use of the product BUD. 97 % of the volunteers found their skins soft or very soft after the use of the product BAE.

Remark: In the group BAE, 2 volunteers were not able to answer because the product was too harsh for them.

*Skin cleaner sensation after washing*

Table 4: Skin cleaner sensation assessment (Questionnaire)

| Type of product | Number of volunteers | % of volunteers |
|---|---|---|
| BAE | 9 | 31 |
| BUD | 11 | 38 |
| No difference | 9 | 31 |

Most volunteers (38%) found their skins cleaner with the product BUD in comparison to the product BAE (31%).

*Product preference*

Table 5: Skin cleaner sensation assessment (Questionnaire)

| Type of product | Number of volunteers | % of volunteers |
|---|---|---|
| BAE | 6 | 21 |
| BUD | 23 | 79 |
| No difference | 0 | 0 |

The majority of the volunteers (79%) were satisfied by BUD product and preferred BUD product to BAE product. The volunteers found the BUD product very easy to apply and to use. They were satisfied because they had a new experience sensation. The product were less aggressive for their skins with a high efficacy.

Tolerance properties

*Sensation of skin aggression (feeling of scratches) by exfoliating particle*

Table 6: Assessment of skin sensation of aggression (Questionnaire)

| Type of product | Number of volunteers | % of volunteers |
|---|---|---|
| BAE | 29 | 100 |
| BUD | 0 | 0 |
| No difference | 0 | 0 |

All the volunteers (100%) felt scratching sensation of their skins with the BAE product. With the BUD product, any aggression was felt.

*Skin redness and/or slight irritation (after intensive use)*

Table 7: Assessment of skin redness and/or slight irritation (Questionnaire)

| Type of product | Number of volunteers | % of volunteers |
|---|---|---|
| BAE | 21 | 84 |
| BUD | 1 | 4 |
| None | 2 | 8 |
| Both | 1 | 4 |

84% of the volunteers showed skin irritation with the fruit kernel fragment particles, whereas only 4% mentioned skin irritation with the cellulose particles.

Remark: 4 volunteers didn't answer to the questionnaire.

**Conclusions**

The clinical investigation has shown that both products assessed were efficient exfoliators and confirms the efficacy of the composition of the invention seen during ex vivo experiments. The cream of the invention improved the following clinical parameters:

Softness: composition of the invention (100%) versus apricot exfoliator BAE (97%)
Cleanness: composition of the invention (38%) versus apricot exfoliator BAE (31%)
Aggressiveness: composition of the invention (0%) versus apricot exfoliator BAE (100%)
Redness or slight irritation: composition of the invention (0%) versus apricot exfoliator BAE (84%)

*Experimentation 4: Assessment of the spreadable and disintegrative properties*

To determine the average "play time" of cellulose particles in cosmetic composition during application a panel test has been performed.
The "play time" parameter was assessed based on total time that was required to completely destroy cellulose particles on the skin (i.e. 100% of the cellulose particles present on the skin were destroyed).

**Material & method**

*Products used:*

Sample 1: Gel formula (see experimentation 2 for the gel formula) containing 3% cellulose particles fraction of 0 - 200 $\mu$m with an average size of 100 $\mu$m;
Sample 2: Gel formula (see experimentation 2 for the gel formula) containing 3% cellulose particles fraction of 200 - 400 $\mu$m with an average size of 300 $\mu$m;
Sample 3: Gel formula (see experimentation 2 for the gel formula) containing 3% cellulose particles fraction of 400 - 900 $\mu$m with an average size of 650 $\mu$m;
Sample 4: Gel formula (see experimentation 2 for the gel formula) containing 5% cellulose particles fraction of 400 - 900 $\mu$m with an average size of 650 $\mu$m;

*Panel and study conditions:*

A panel test was run on 20 people to determine the average time required to completely destroy cellulose particles on the outer surface of a hand.

The study was simple blinded. Each sample was tested ones by each volunteer.

### Results

The average "play time" assessment showed the following results:

Sample 1: in average 12 seconds until particles are completely destroyed
Sample 2: in average 20 seconds until particles are completely destroyed
Sample 3: in average 30 seconds until particles are completely destroyed
Sample 4: in average 42 seconds until particles are completely destroyed

### Conclusions

The results of the panel test showed that the "play time" parameter depends not only on the size of cellulose particles, but also on the concentration in gel. The larger the particles and the higher the concentration the longer it is to completely destroy cellulose particles.

### EXAMPLES

*Example 1*

An intensive face peeling cream for mature skin was prepared with Unisteron Y-50 and cellulose particles as described below (all quantities being given in wt% or wt parts on product as mentioned):

Table 8: Composition of the intensive face peeling cream

| Part | Trade Name | INCI Name | % w/w |
|---|---|---|---|
| A | Water | Aqua | ad. 100.00 |
| | Glycerin | Glycerin | 2.00 |
| | Lara Care A 200 | Galactoarabinan | 0.20 |
| | Butylene Glycol | Butylene Glycol | 3.00 |
| A 1 | Carbopol ETD 2050 | Carbomer | 0.15 |
| | Carbopol ETD 2001 | Carbomer | 0.10 |
| | | | |
| B | Brij 721 | Steareth-21 | 2.50 |
| | Arlacel 161 | Glyceryl Stearate | 2.50 |
| | Lanette O | Cetearyl Alcohol | 0.50 |
| | Myritol 318 | Caprylic Capric Triglyceride | 7.00 |
| | Cegesoft PS 6 | Vegetable Oil / Olus | 5.00 |
| | Estol 3609 | Triethylhexanoin | 1.00 |
| | Prisorine 2021 | Isopropyl Isostearate | 2.00 |
| | Dow Corning 2503 | Stearyl Dimethicone | 1.00 |
| | | | |
| B 1 | Dow Corning 245 | Cyclopentasiloxane | 1.00 |
| | | | |
| C | Neutrol TE 50 % | Tetrahydoxypropyl Ethylenediamine, Aqua | approx. 0.60 |
| | | | |

(continued)

| Part | Trade Name | INCI Name | % w/w |
|------|-----------|-----------|-------|
| D | Unisteron Y-50 | Oleyl Alcohol, Dioscorea Villosa (Wild Yarn) Root Extract, Glycine Soja (Soybean) Sterols | 5.00 |
| | Uniphen P-23 | Phenoxyethanol, Methylparaben, Ethylparaben, Butylparaben, Propylparaben, Isobutylparaben | 1.00 |
| | Parfum High Society 05 | Parfum | 0.30 |
| E | Cellulose particles of the invention with an average size ranging between 0$\mu$m and 200$\mu$m | Cellulose, hydroxypropyl methylcellulose | 1.00-10% |
| pH value: 6 - 6.5 | | | |

Procedure:

1. Blend Part A, then disperse A 1 with stirring till homogenized and heat up to 75-80°C
2. Blend together Part B and heat up to 70-75°C and add B 1
3. Add Part B/B1 slowly with stirring in Part A/A1
4. Neutralize with Part C
5. Homogenize for about 3 minutes
6. Cool with stirring down to approx. 40°C and add Part D and E
7. Cool with stirring down to approx. 27°C

Appearance : soft O/W Cream with particles

_Example 2_

An exfoliating anti-dandruff shampoo was prepared with the following composition (all quantities being given in wt% or wt parts on product as mentioned):

Table 9: Composition of the exfoliating anti-dandruff shampoo

| Part | Trade Name | INCI Name | % w/w |
|------|-----------|-----------|-------|
| A | Water | Aqua | ad 100.0 |
| | Unidiacide U-26 | Diazolidinyl Urea | 0.20 |
| A1 | Ucare IR-400 | Polyquaternium-10 | 0.30 |
| | | | |
| B | Tego Betain F 50 | Cocamidopropyl Betaine | 8.00 |
| | Plantacare 1200 | Lauryl Glucoside | 3.00 |
| | Rewoteric AMC | Sodium Cocoamphoacetate | 2.00 |
| | | | |
| B1 | Cellulose particles of the invention with an average size ranging between 200$\mu$m and 400$\mu$m | Cellulose, hydroxypropyl methylcellulose | 5.00. |
| | | | |
| B2 | Zetesol LA-2 (28%) | Ammonium Laureth Sulfate | 20.00 |
| | | | |

(continued)

| Part | Trade Name | INCI Name | % w/w |
|------|-----------|-----------|-------|
| C | Fragrance | Parfum | 0.50 |
| | | | |
| D | DL-Panthenol 50 W | Panthenol; Aqua | 0.50 |
| | | | |
| | Unicat MM | Methylchloroisothiazolinone; Methylisothiazolinone | 0.10 |
| | | | |
| F | Antil 200 | PEG-200 Hydrogenated Glyceryl Palmate; PEG-7 Glyceryl Cocoate | q.s. |
| pH-Value 5.5 - 6.5 Viscosity 4 000 - 6 000 cP | | | |

Procedure:

1. Mix Part A, add A1 with stirring to clear viscous solution
2. Mix Part B with stirring
3. Add Part B 1
4. Add Part B2 with stirring
5. Dissolve Part C in B/B1/B2 with stirring
6. Add Part A/A1 to Part B/C with stirring
7. Add Part D and E with stirring
8. Adjust pH-Value and Viscosity, if necessary

Appearance: clear shampoo with Particles

*Example 3*

An exfoliating anti-cellulite cream was prepared with Unilactamin L-17 and cellulose particles of the invention as described below (all quantities being given in wt% or wt parts on product as mentioned):

Table 10: Composition of the anti-cellulite cream

| Part | Trade Name | INCI Name | % w/w |
|------|-----------|-----------|-------|
| A | Water | Aqua | ad.100.00 |
| | Glycerin | Glycerin | 2.00 |
| | Butylene Glycol | Butylene Glycol | 3.00 |
| A1 | Carbopol ETD 2020 | Acrylates/C 10-30 Alkyl Acrylate Crosspolymer | 0.20 |
| | Carbopol Ultrez 10 | Carbomer | 0.10 |
| | | | |
| B | Brij S721 | Steareth-21 | 2.50 |
| | Cutina GMS | Glyceryl Stearate | 2.50 |
| | Lanette O | Cetearyl Alcohol | 0.50 |
| | Myritol 312 | Caprylic Capric Triglyceride | 7.00 |
| | Cegesoft PS 6 | Vegetable Oil / Olus | 5.00 |
| | Estol 3609 | Triethylhexanoin | 1.00 |

(continued)

| Part | Trade Name | INCI Name | % w/w |
|------|-----------|-----------|-------|
| | Prisorine 2021 | Isopropyl Isostearate | 2.00 |
| | Dow Corning 2503 Cosmetic Wax | Stearyl Dimethicone | 1.00 |
| | Cellulose particles of the invention with an average size ranging between 400μm and 900μm | Cellulose, hydroxypropyl methylcellulose | 1.0 - 5.0 |
| B1 | Xiameter(R) PMX-0245 Cyclosiloxane | Cyclopentasiloxane | 1.00 |
| | | | |
| C | Neutrol TE 50% | Tetrahydroxypropyl Ethylenediamine | app. 0.60 |
| | | | |
| D | Unilactamin L-17 | Butylene Glycol, Hydrolyzed Milk Protein, Niacinamide, Adenosine Triphosphate | 3.00 |
| | Uniphen P-23 | Phenoxyethanol, Methylparaben, Ethylparaben, Butylparaben, Propylparaben, Isobutylparaben | 1.00 |
| | | | |
| E | Fragrance | Parfum | q.s. |
| pH value: 6 - 6.5 | | | |

Procedure:

1 Blend Part A, then disperse A1 with stirring till homogenized and heat up to 70-75°C
2 Blend together Part B and heat up to 70-75°C, and add Part B 1
3 Add Part B/B1 slowly with stirring to Part A/A1
4 Neutralize with Part C.
5 Homogenize for about 3 minutes.
6 Cool with stirring to approx. 40°C, then add Part D and E
7 Homogenize shortly, and then cool with stirring to approx. 27°C

Appearance: white soft O/W cream

*Example 4*

A deep moisturizing cleansing lotion for oily skin was prepared with the following composition (all quantities being given in wt% or wt parts on product as mentioned):

Table 11: Composition of the deep moisturizing cleansing lotion

| Part | Trade Name | INCI Name | % w/w |
|------|-----------|-----------|-------|
| A | Water | Aqua | ad. 100.00 |
| | Butylene Glycol | Butylene Glycol | 3.00 |
| A 1 | Carbopol Ultrez 21 | Acrylates/C10-30 Acrylate Crosspolymer | 0.25 |
| | | | |
| B | Brij 721 | Steareth-21 | 2.50 |
| | Arlacel 161 | Glyceryl Stearate | 2.50 |

(continued)

| Part | Trade Name | INCI Name | % w/w |
|------|-----------|-----------|-------|
| | Lanette O | Cetearyl Alcohol | 0.60 |
| | Miglyol 318 | Caprylic/Capric Triglyceride | 4.00 |
| | Eutanol G | Octyldodecanol | 3.00 |
| | Cetiol MM | Myristyl Myristate | 1.00 |
| | Dow Corning 200/100 | Dimethicone | 1.00 |
| | | | |
| B 1 | Dow Corning 345 | Cyclopentasiloxane, Cyclohexasiloxane | 3.00 |
| | | | |
| C | Sodium Hydroxide 18% | Sodium Hydroxide/Aqua | approx. 0.30 |
| | | | |
| D | Uniphen P-23 | Phenoxyethanol, Methylparaben, Ethylparaben, Butylparaben, Propylparaben, Isobutylparaben | 1.00 |
| | Cellulose particles of the invention with an average size ranging between 0$\mu$m and 200 $\mu$m | Cellulose, hydroxypropyl methylcellulose | 5.00 |
| E | Fragrance | Parfum | 0.30 |
| pH value: 6 - 6.5 | | | |

Procedure:

1. Blend Part A, then disperse A 1 with stirring till homogenized and heat up to 75°C.
2. Blend together Part B and heat up to 70-75°C and add B 1.
3. Add Part B/B1 slowly with stirring in Part A/A1
4. Homogenize for about 3 minutes.
5. Neutralize with Part C.
6. Cool with stirring to approx. 40°C and add Part D and Part E.
7. Cool with stirring to approx. 27°C.

Appearance: white O/W cream

*Example 5*

An intensive face peeling cream was prepared with Unisteron Y-50 and cellulose particles of the invention as described below (all quantities being given in wt% or wt parts on product as mentioned):

Table 12: Composition of the intensive face peeling cream

| Part | Trade Name | INCI Name | % w/w |
|------|-----------|-----------|-------|
| A | Water | Aqua | ad. 100.00 |
| | Glycerin | Glycerin | 2.00 |
| | Lara Care A 200 | Galactoarabinan | 0.20 |
| | Butylene Glycol | Butylene Glycol | 3.00 |
| A 1 | Carbopol ETD 2050 | Carbomer | 0.15 |

(continued)

| Part | Trade Name | INCI Name | % w/w |
|------|------------|-----------|-------|
| | Carbopol ETD 2001 | Carbomer | 0.10 |
| | | | |
| B | Brij 721 | Steareth-21 | 2.50 |
| | Arlacel 161 | Glyceryl Stearate | 2.50 |
| | Lanette O | Cetearyl Alcohol | 0.50 |
| | Myritol 318 | Caprylic Capric Triglyceride | 7.00 |
| | Cegesoft PS 6 | Vegetable Oil / Olus | 5.00 |
| | Estol 3609 | Triethylhexanoin | 1.00 |
| | Prisorine 2021 | Isopropyl Isostearate | 2.00 |
| | Dow Corning 2503 | Stearyl Dimethicone | 1.00 |
| | | | |
| B 1 | Dow Corning 245 | Cyclopentasiloxane | 1.00 |
| | | | |
| C | Neutrol TE 50 % | Tetrahydoxypropyl Ethylenediamine, Aqua | approx. 0.60 |
| | | | |
| D | Unisteron Y-50 | Oleyl Alcohol, Dioscorea Villosa (Wild Yarn) Root Extract, Glycine Soja (Soybean) Sterols | 5.00 |
| | Uniphen P-23 | Phenoxyethanol, Methylparaben, Ethylparaben, Butylparaben, Propylparaben, Isobutylparaben | 1.00 |
| | Parfum High Society 05 | Parfum | 0.30 |
| E | Cellulose particles of the invention with an average size ranging between 200$\mu$m and 400$\mu$m | Cellulose, hydroxypropyl methylcellulose | 1.00 - 10% |
| pH value: 6 - 6.5 | | | |

Procedure:

1. Blend Part A, then disperse A 1 with stirring till homogenized and heat up to 75-80°C
2. Blend together Part B and heat up to 70-75°C and add B 1
3. Add Part B/B1 slowly with stirring in Part A/A1
4. Neutralize with Part C
5. Homogenize for about 3 minutes
6. Cool with stirring down to approx. 40°C and add Part D
7. Cool with stirring down to approx. 27°C

Appearance: soft O/W Cream with particles

*Example 6*

A natural beauty body cleaning gel was prepared with Unitamuron H-22, Unigard OA-94 and the cellulose particles of the invention as described below (all quantities being given in wt% or wt parts on product as mentioned):

Table 13: Composition of the body cleaning gel

| Part | Trade Name | INCI Name | % w/w |
|------|-----------|-----------|-------|
| A | Water | Aqua / Water | ad 100 |
| | Glycerin min. 99% | Glycerin | 7.00 |
| | Aloe Vera 100 | Aloe Barbadensis Leaf Extract | 0.20 |
| | | | |
| A1 | Keltrol T | Xanthan Gum | 0.70 |
| | Genuvisco X-923-03 | Carrageenan | 0.25 |
| B | Rose Water | Rosa Centifolia Flower Water | 10.00 |
| | Hamamelis Water | Hamamelis Virginiana (Witch Hazel) Leaf Water | 3.00 |
| | D-PTA | Panthenol Triacetate | 1.00 |
| | Cellulose particles of the invention with an average size ranging between 400$\mu$m and 900$\mu$m | Cellulose, hydroxypropyl methylcellulose | 3.00 |
| | Unitamuron H-22 | Pentylene Glycol, Tamarindus Indica Seed Gum | 2.00 |
| | | | |
| C | Unigard OA-94 | Phenoxyethanol; Benzoic Acid; Dehydroacetic Acid | 1.00 |
| D | Water | Aqua / Water | 3.00 |
| | Citric Acid Monohydrate | Citric Acid | 0.02 |
| | Trisodium Citrate Hydrate | Sodium Citrate | 0.08 |
| pH value: 5 - 5.5 | | | |

Procedure:

1. Blend Part A and stir to clear solution
2. Add Part A1 and stir until homogenous
3. Blend Part B with stirring
4. Add Part B to Part A/A1 with stirring to form a homogenous viscous gel
5. Add Part C slowly with stirring
6. Adjust pH-value with Part D: Citric Acid / Sodium Citrate solution

Appearance: transparent gel with particles

*Example 7*

A natural body massage gel was prepared with D-PTA, Unitamuron H-22, Unisurrection S-61, Unigard OA-94 and cellulose particles of the invention, as described below (all quantities being given in wt% or wt parts on product as mentioned):

Table 14: Composition of the body massage gel

| Part | Trade Name | INCI Name | % w/w |
|------|-----------|-----------|-------|
| A | Water | Aqua / Water | ad 100 |

(continued)

| Part | Trade Name | INCI Name | % w/w |
|------|-----------|-----------|-------|
| | Glycerin min. 99% | Glycerin | 7.00 |
| | Aloe Vera 100 | Aloe Barbadensis Leaf Extract | 0.20 |
| | | | |
| A1 | Keltrol T | Xanthan Gum | 0.70 |
| | Genuvisco X-923-03 | Carrageenan | 0.25 |
| | Cellulose particles of the invention with an average size ranging between 200μm and 400μm | Cellulose, hydroxypropyl methylcellulose | |
| B | Rose Water | Rosa Centifolia Flower Water | 10.00 |
| | Hamamelis Water | Hamamelis Virginiana (Witch Hazel) Leaf Water | 3.00 |
| | D-PTA | Panthenol Triacetate | 1.00 |
| | Unisurrection S-61 | Beta Vulgaris (Beet) Root Extract, Glycerin, Haberlea Rhodopensis Leaf Extract, Faex (Yeast) Extract | 3.00 |
| | Unitamuron H-22 | Pentylene Glycol, Tamarindus Indica Seed Gum | 2.00 |
| | | | |
| C | Unigard OA-94 | Phenoxyethanol; Benzoic Acid; Dehydroacetic Acid | 1.00 |
| D | Water | Aqua / Water | 3.00 |
| | Citric Acid Monohydrate | Citric Acid | 0.02 |
| | Trisodium Citrate Hydrate | Sodium Citrate | 0.08 |
| pH value: 5 - 5.5 | | | |

Procedure:

1. Blend Part A and stir to clear solution
2. Add Part A1 and stir until homogenous
3. Blend Part B with stirring
4. Add Part B to Part A/A1 with stirring to form a homogenous viscous gel
5. Add Part C slowly with stirring
6. Adjust pH-value with Part D: Citric Acid / Sodium Citrate solution

Appearance: Transparent gel

_Example 8_

A peeling body shampoo was prepared with the following composition (all quantities being given in wt% or wt parts on product as mentioned):

Table 15: Composition of the peeling body shampoo

| Part | Trade Name | INCI Name | % w/w |
|------|-----------|-----------|-------|
| A | Water | Aqua | ad. 100.00 |
| | Carbopol Aqua SF-1 | Acrylates Copolymer | 10.00 |

(continued)

| Part | Trade Name | INCI Name | % w/w |
|------|-----------|-----------|-------|
| | Unicide U-13 | Imidazolidinyl Urea | 0.30 |
| B | Texapon NSO BZ | Sodium Laureth Sulfate | 25.00 |
| | Dehyton PK 45 | Cocamidopropyl Betaine | 4.00 |
| | Plantacare 818 UP | Coco Glucoside | 4.00 |
| | Fragrance | Parfum | q.s. |
| | | | |
| C | Sodium Hydroxide 18% | Sodium Hydroxide | app. 1.50 |
| | | | |
| D | Cellulose particles of the invention with an average size ranging between 400µm and 900µm | Cellulose, hydroxypropyl methylcellulose | 1,0 - 5.0 |
| pH value: 6 - 6.5 | | | |

Procedure:

1. Blend Part A under slowly stirring till homogenized
2. Blend Part B with stirring to homogenous mixture
3. Add Part A slowly with stirring to Part B
4. Neutralize with Part C.
5. Add Part D under slowly stirring.

Appearance: clear viscous Shampoo with particles

**Claims**

1. Cellulose particles having an average particle size less than or equal to 2 mm, wherein the cellulose particles provide measurable exfoliation after 1 day in a cosmetic composition, preferably after 3 months in a cosmetic composition, more preferably after 6 months in a cosmetic composition, even more preferably after 12 months in a cosmetic composition.

2. The cellulose particles of claim 1, wherein at least 80%, preferably at least 90%, more preferably at least 95%, and even more preferably at least 99% of the cellulose particles after 1 day in a cosmetic composition, disintegrate upon applying on human skin or scalp.

3. The cellulose particles of any of the previous claims, wherein said cellulose particles further contain at least one cosmetic agent and/or color agent, preferably a liposoluble agent.

4. The cellulose particles of claim 3, wherein the cosmetic/color agent is chosen among vitamins, coenzymes, essential oils, natural oils, silicone oils, clay or metal or precious stone powder, D-panthenyl triacetate, pigments.

5. Use of cellulose particles according to any of claims 1 to 4, for the preparation of a body care composition, in particular cleansing body composition, hair care composition and cosmetic composition.

6. Cosmetic use of cellulose particles according to any one of claims 1 to 4, as exfoliating agents for skin or scalp.

7. The cosmetic use of claim 6, to exfoliate during cleansing of the skin or scalp of the body or to exfoliate part of body skin or scalp without rinsing out.

**8.** A body care composition, comprising cellulose particles according to any one of claims 1 to 4.

**9.** The body care composition according to claim 8, wherein the cellulose particles provide measurable exfoliation after 3 months storage at room temperature, preferably after 6 months, and more preferably after 12 months storage at room temperature.

**10.** The body care composition of claim 8 or 9, wherein at least 80%, preferably at least 90%, more preferably at least 95%, and even more preferably at least 99% of the cellulose particles disintegrate after an average playtime less than 3 minutes, preferably less than 2 minutes and more preferably less than 1 minutes, when applied on human skin or scalp.

**11.** The body care composition according to any one of claims 8 to 10, which comprises 0.5% to 20% by wt. of said cellulose particles, preferably 1% to 15% by wt., more preferably 2% to 20% by wt., even more preferably 3% to 5% by wt.

**12.** The body care composition according to any of claims 8 to 11, wherein the distribution size of cellulose particles ranges between 0 and 200$\mu$m, or between 200 and 400 $\mu$m, or between 400 and 900 $\mu$m.

**13.** The body care composition according to any of claims 8 to 12, wherein the body care composition is a cleansing body composition, in particular a peeling-shower gel composition, hair care composition, in particular a peeling-shampoo, or a cosmetic composition, in particular exfoliating gels, whitening and/or brightening composition, body and feet scrub composition and/or wherein the composition is for preventing and/or reversing the sign of skin aging, improving skin smoothness, and/or recovering skin moisturization, and/or preventing trans-epidermal water loss in skin.

**14.** The body care composition according to any of claims 8 to 13, further comprising at least one additive selected from the group consisting of excipients, vehicle ingredients, moisturizers, humectants, cosmetic salts, adjuvants, oils, emulsifiers, co-emulsifiers, gelling agents, absorbers, solvents, photoprotective agents, and inert base.

**15.** A cosmetic method of exfoliating skin or scalp, comprising applying to said skin or scalp a composition as claimed in any of claims 8 to 14.

FIGURE 1

FIGURE 1 (CONT.)

EP 2 907 498 A1

FIGURE 1 (CONT.)

2A                                    2B                                    2C

FIGURE 2

Europäisches
Patentamt

European
Patent Office

Office européen
des brevets

# EUROPEAN SEARCH REPORT

Application Number

EP 14 30 5185

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2012/034282 A1 (BOYD THOMAS [US] ET AL) 9 February 2012 (2012-02-09) * claims; examples; table 1 * | 1-15 | INV. A61K8/73 A61Q19/00 A61K8/02 |
| X | DE 44 03 710 A1 (GOLDWELL GMBH [DE]) 10 August 1995 (1995-08-10) * claims; examples * | 1-15 | |
| X | JP S63 238008 A (DAICEL CHEM) 4 October 1988 (1988-10-04) * abstract * | 1-15 | |
| X | DATABASE GNPD [Online] MINTEL; 30 November 2010 (2010-11-30), "Soft Gel", XP002726963, Database accession no. 1424140 * the whole document * | 1-15 | |
| X | US 2012/277137 A1 (IKEGAKI SHINICHI [JP] ET AL) 1 November 2012 (2012-11-01) * examples 1-16, 18-19, 26-27 * | 1-15 | TECHNICAL FIELDS SEARCHED (IPC) A61K A61Q |
| X | EP 0 829 259 A1 (WARNER LAMBERT CO [US]) 18 March 1998 (1998-03-18) * claims * | 1-15 | |
| X | DE 10 2004 038771 A1 (HENKEL KGAA [DE]) 4 August 2005 (2005-08-04) * claims; examples * | 1-15 | |
| X | US 2006/270563 A1 (YANG HUI S [US] ET AL YANG HUI SHIRLEY [US] ET AL) 30 November 2006 (2006-11-30) * paragraph [0171] * | 1-15 | |

-/--

The present search report has been drawn up for all claims

| Place of search | Date of completion of the search | Examiner |
|---|---|---|
| Munich | 15 July 2014 | Miller, Bernhard |

Europäisches
Patentamt

European
Patent Office

Office européen
des brevets

# EUROPEAN SEARCH REPORT

Application Number

EP 14 30 5185

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| E | WO 2014/074578 A1 (JOHNSON & JOHNSON CONSUMER [US]) 15 May 2014 (2014-05-15) * claims; examples * ----- | 1-15 | |
| X | DE 197 17 828 A1 (WARTENBERG ARNOLD PROF DR [DE]) 29 October 1998 (1998-10-29) * claims 1,8; examples * ----- | 1-5,8-14 | |
| X | US 2005/113267 A1 (POPPLEWELL LEWIS M [US] ET AL POPPLEWELL LEWIS MICHAEL [US] ET AL) 26 May 2005 (2005-05-26) * example II(A) * ----- | 1-4 | |
| X | US 5 725 884 A (SHERWOOD BOB E [US] ET AL) 10 March 1998 (1998-03-10) * claims 2,12 * ----- | 1-4 | |
| X | "Unispheres MANNITOL (LACTOSE) SERIES, NATURAL SERIES AND PRECIOUS COLLECTION MACROSCOPIC CARRIER BEADS, CONTAINING PIGMENTS, ACTIVE INGREDIENTS AND/OR GEMSTONES, FOR COSMETIC PRODUCTS", , 20 December 2012 (2012-12-20), pages 1-10, XP055128187, Retrieved from the Internet: URL:https://web.archive.org/web/2012122005 4216/http://www.in-cosmetics.com/__novadoc uments/2577 [retrieved on 2014-07-10] * the whole document * ----- | 1-5,8-14 | TECHNICAL FIELDS SEARCHED (IPC) |

The present search report has been drawn up for all claims

| Place of search | Date of completion of the search | Examiner |
|---|---|---|
| Munich | 15 July 2014 | Miller, Bernhard |

EPO FORM 1503 03.82 (P04C01)

## ANNEX TO THE EUROPEAN SEARCH REPORT
## ON EUROPEAN PATENT APPLICATION NO.

EP 14 30 5185

This annex lists the patent family members relating to the patent documents cited in the above-mentioned European search report.
The members are as contained in the European Patent Office EDP file on
The European Patent Office is in no way liable for these particulars which are merely given for the purpose of information.

15-07-2014

| Patent document cited in search report | | Publication date | Patent family member(s) | | Publication date |
|---|---|---|---|---|---|
| US 2012034282 | A1 | 09-02-2012 | AR | 076048 A1 | 11-05-2011 |
| | | | AU | 2009343764 A1 | 22-09-2011 |
| | | | CA | 2757117 A1 | 07-10-2010 |
| | | | CN | 102378621 A | 14-03-2012 |
| | | | EP | 2413884 A1 | 08-02-2012 |
| | | | JP | 2012522779 A | 27-09-2012 |
| | | | RU | 2011144354 A | 10-05-2013 |
| | | | SG | 173787 A1 | 29-09-2011 |
| | | | TW | 201102100 A | 16-01-2011 |
| | | | US | 2012034282 A1 | 09-02-2012 |
| | | | WO | 2010114549 A1 | 07-10-2010 |
| DE 4403710 | A1 | 10-08-1995 | NONE | | |
| JP S63238008 | A | 04-10-1988 | NONE | | |
| US 2012277137 | A1 | 01-11-2012 | CN | 102647975 A | 22-08-2012 |
| | | | EP | 2510917 A1 | 17-10-2012 |
| | | | JP | 2011140485 A | 21-07-2011 |
| | | | TW | 201127416 A | 16-08-2011 |
| | | | US | 2012277137 A1 | 01-11-2012 |
| | | | WO | 2011071079 A1 | 16-06-2011 |
| EP 0829259 | A1 | 18-03-1998 | EP | 0829259 A1 | 18-03-1998 |
| | | | JP | H1095720 A | 14-04-1998 |
| DE 102004038771 | A1 | 04-08-2005 | DE | 102004038771 A1 | 04-08-2005 |
| | | | EP | 1634568 A1 | 15-03-2006 |
| US 2006270563 | A1 | 30-11-2006 | AR | 057044 A1 | 14-11-2007 |
| | | | EP | 1885679 A2 | 13-02-2008 |
| | | | EP | 2484657 A2 | 08-08-2012 |
| | | | EP | 2495232 A2 | 05-09-2012 |
| | | | ES | 2389776 T3 | 31-10-2012 |
| | | | US | 2006270563 A1 | 30-11-2006 |
| | | | US | 2010273683 A1 | 28-10-2010 |
| | | | US | 2012116005 A1 | 10-05-2012 |
| | | | US | 2012116040 A1 | 10-05-2012 |
| | | | US | 2012121523 A1 | 17-05-2012 |
| | | | US | 2012123149 A1 | 17-05-2012 |
| | | | US | 2013129647 A1 | 23-05-2013 |
| | | | US | 2013296516 A1 | 07-11-2013 |
| | | | US | 2014099276 A1 | 10-04-2014 |
| | | | WO | 2006130675 A2 | 07-12-2006 |
| WO 2014074578 | A1 | 15-05-2014 | US | 2014134217 A1 | 15-05-2014 |

For more details about this annex : see Official Journal of the European Patent Office, No. 12/82

## ANNEX TO THE EUROPEAN SEARCH REPORT
## ON EUROPEAN PATENT APPLICATION NO.                     EP 14 30 5185

This annex lists the patent family members relating to the patent documents cited in the above-mentioned European search report.
The members are as contained in the European Patent Office EDP file on
The European Patent Office is in no way liable for these particulars which are merely given for the purpose of information.

15-07-2014

| Patent document cited in search report | Publication date | Patent family member(s) | Publication date |
|---|---|---|---|
| | | WO 2014074578 A1 | 15-05-2014 |
| DE 19717828 A1 | 29-10-1998 | NONE | |
| US 2005113267 A1 | 26-05-2005 | DE 602004012820 T2 | 07-05-2009 |
| | | EP 1533365 A1 | 25-05-2005 |
| | | US 2005113267 A1 | 26-05-2005 |
| US 5725884 A | 10-03-1998 | AT 239450 T | 15-05-2003 |
| | | AT 441403 T | 15-09-2009 |
| | | AT 445391 T | 15-10-2009 |
| | | AU 698667 B2 | 05-11-1998 |
| | | BR 9605329 A | 16-09-1997 |
| | | CA 2183881 A1 | 18-07-1996 |
| | | DE 69627934 D1 | 12-06-2003 |
| | | DE 69627934 T2 | 12-02-2004 |
| | | DK 0752848 T3 | 01-09-2003 |
| | | EP 0752848 A1 | 15-01-1997 |
| | | EP 1287823 A1 | 05-03-2003 |
| | | ES 2199281 T3 | 16-02-2004 |
| | | FI 963497 A | 06-11-1996 |
| | | HU 9602360 A2 | 28-08-1997 |
| | | IL 116675 A | 31-10-2000 |
| | | JP 3300364 B2 | 08-07-2002 |
| | | JP H10500426 A | 13-01-1998 |
| | | NO 963732 A | 08-11-1996 |
| | | PT 752848 E | 29-08-2003 |
| | | US 5585115 A | 17-12-1996 |
| | | US 5725883 A | 10-03-1998 |
| | | US 5725884 A | 10-03-1998 |
| | | US 6103219 A | 15-08-2000 |
| | | US 6217909 B1 | 17-04-2001 |
| | | US 2001001664 A1 | 24-05-2001 |
| | | US 2002142032 A1 | 03-10-2002 |
| | | US 2003096005 A1 | 22-05-2003 |
| | | US 2005013861 A1 | 20-01-2005 |
| | | US 2009169621 A1 | 02-07-2009 |
| | | WO 9621429 A1 | 18-07-1996 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 0850642 A

- WO 9800094 A

**Non-patent literature cited in the description**

- Remington's Pharmaceutical Sciences. Mack Publishing Co, 1990